(19) Europäisches Patentamt
European Patent Office
Office européen des brevets

(11) Publication number: 0 092 756
B1

(12) EUROPEAN PATENT SPECIFICATION

(45) Date of publication of patent specification: 24.06.87

(21) Application number: 83103707.2

(22) Date of filing: 16.04.83

(51) Int. Cl.⁴: C 07 F 7/00, C 08 J 3/02 // E21B33/138, E21B43/25, C08L1/08, C08L5/00

# (54) Zirconate thickening agents.

(30) Priority: 22.04.82 US 371024

(43) Date of publication of application: 02.11.83 Bulletin 83/44

(45) Publication of the grant of the patent: 24.06.87 Bulletin 87/26

(84) Designated Contracting States: AT BE DE FR GB IT NL SE

(56) References cited:
EP-A-0 073 502
GB-A-1 487 794
US-A-3 474 069

JOURNAL OF CHEMICAL SOCIETY, 1970, U.B. SAXENA et al. "Reactions of zirconium isopropoxide with bêta-diketones and bêta-keto-esters", pages 904-7

CHEMICAL ABSTRACTS, volume 79, no. 4, July 30, 1973, page 152, abstract 21119s (COLUMBUS OHIO, US) & JP - A - 73 04 771 (MATSUMOTO SEIYAKU KOGYO CO.) 10-02-1973

(73) Proprietor: KAY-FRIES, Inc.
Stony Point New York 10980 (US)

(72) Inventor: Rummo, Gregory J.
51 Cross Street Apt. 2D
Bronxville New York 10708 (US)

(74) Representative: Siecke, Wolf-Friedrich, Dr. et al
c/o DYNAMIT NOBEL AKTIENGESELLSCHAFT
Patentabteilung Postfach 1261
D-5210 Troisdorf/Bez. Köln (DE)

(56) References cited:
CHEMICAL ABSTRACTS, volume 73, no. 18, November 2, 1970, page 469, abstract 94204b (COLUMBUS, OHIO, US) D.M. PURI "Organic derivatives of zirconium. I. Reactions with bidentate ligands", & J. Indian. Chem. SOC. 1970, 47(6), 535-40

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 092 756 B1

**Description**

This invention relates to the use of the reaction products of alkyl and/or aryl zirconates and ß-diketones as thickeners for fluids used in resource recovery operations, such as by incorporating the product zirkonates described herein in drilling muds, consolidation fluids or fracturing fluids.

According to the invention, the product zirconates, which are obtained from tetraalkyl and/or tetraaryl zirconates and ß-diketones at rom temperature, when utilized as described herein as thickening agents for fluids used in resource recovery operations, *e.g.,* aqueous solutions of hydroxypropylguar (HPG), effect an increase in the viscosity of the fluids.

References pertinent to an understanding of the background of the invention include (1) Puri, "Organic Derivatives of Zirconium. I. Reactions with Bidentate Ligands", 47 J. Indian Chem. Soc. 535—540 (1970); (2) Saxena *et al.,* "Reactions of Zirconium Isopropoxide with Beta Diketones and Beta Keto Esters", 47 J. Chem. Soc. 904—907 (1970); (3) Sugiyama *et al.,* Japanese Patent No. 75 05747; and (4) Sugiyama *et al.,* Japanese Patent No. 73 04771.

In Reference (1) above, Puri discloses that acetylacetone reacts with tetra (isopropyl)zirconate in different molar ratios to yield various products, including Zr(isopropoxy)$_3$(acac)·isopropanol, Zr(isopropoxy)$_2$(acac)$_2$ and Zr(isopropoxy)(acac)$_3$. Saxena *et al.,* in Reference (2) above, disclose the reaction of tetra(isopropyl)zirconate with, *inter alia,* acetylacetone to yield compounds of the type Zr(isopropoxy)$_{4-x}$ L$_x$, where L is, among other things, (acac) and x=1—4. Sugiyama *et al.,* in Reference (3), disclose that acrylic copolymers, *e.g.,* butyl methacrylate-2-hydroxyethyl acrylate polymer were crosslinked with diisopropoxyzirconium bis(acetylacetonate). The same authors, in Reference (4), disclose the refluxing of tetraisopropylzirconate with Zr tetrakis(acetylacetonate) in PhMe for one hour to yield diisopropoxyzirconium bis(acetoxyacetonate).

Processes leading to products of for use in this invention can be carried out between 0°C to 100°C, more preferably between 10°C to 80°C, and most preferably between 25°C and 65°C. The reactants can be any of the tetraalkyl or tetraaryl zirconates with any of the ß-diketones resulting in products according to the formula

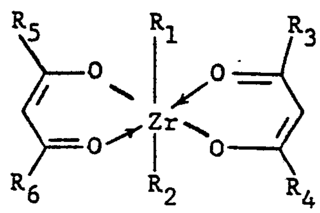

where R$_1$ and R$_2$ may be the same or different and are individually selected from alkoxy having from 1 to 8 carbon atoms, including methoxy, ethoxy, n-propoxy, isopropoxy and the like up to octoxy; aryloxy having from 6 to 12 carbon atoms; and

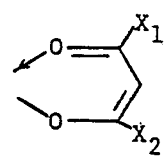 ;

and X$_1$, X$_2$ and R$_3$ to R$_6$ may be the same or different and are individually alkyl having from 1 to 8 carbon atoms, including methyl, ethyl, propyl, isopropyl, n-butyl, tert-butyl, sec-butyl and the like up to octyl, or individually aryl having from 6 to 12 carbon atoms. The most preferable compounds according to the invention have R$_1$ = R$_2$ = n-propoxy or n-butoxy and R$_3$ = R$_4$ = R$_5$ = R$_6$ = methyl.

It has been found that synthesis of these compounds proceeds readily from the admixture of zirconates and ß-diketones in solution at room temperature. Depending on the form of product desired, as explained below, molar ratios of 1:1.5 to 6 (zirconates:ß-diketones) may be employed.

The compounds of this invention are preferably solutions or are maintained as solutions by careful manipulation of solvents and reactant ratios as shown in the following examples and as further explained below.

Example 1

Bis(n-propoxy)bis(2,4-pentanedionato)zirconium(IV)

1121 g tetra(n-propyl)zirconate (70% in n-propanol) were placed in a 3-liter r.b. flask and 481 g acetylacetone added over a 20-minute period. The solution turned orange and increased in temperature to about 50°C. Upon cooling, there was a propensity for the product to crystallize. In order to maintain the product as a liquid, the solution was vacuum distilled (679 mbar at 64°C) and approximately 153 g n-propanol removed. After addition of 400 ml chloroform the product was allowed to cool and has remained stabilized in liquid form at room temperature for several months.

## Example 2

Bis[n-butoxy)bis(2,4-pentanedionato)zirconium(IV)

503.5 g tetra(n-butyl)zirconate (80% in n-butanol) was placed in a 1-liter r.b. flask and 211 g acetylacetone rapidly added. The solution turned orange and increased in temperature to about 60°C. The mixture was allowed to cool to room temperature and showed no propensity to form crystals. Hence unlike with the product of *Example 1,* no stabilizing solvent was required.

## Example 3

922.7 g tetra(n-propyl)zirconate (70% in n-propanol) were placed in a 3 neck r.b. flask. 395.5 g acetylacetone were added dropwise causing a temperature rise to 50°C. The mixture was warmed briefly to 55°C to solvate all solids then allowed to cool. A propensity for the product to crystallize at room temperature was noted.

## Example 4

449.6 g acetylacetone were added to 858.9 g tetra(n-butyl)zirconate (80% in n-butanol) with mixing and warming to 55—60°C. On the third day solids formed in the product.

## Example 5

The products of *Examples 1* and *2* were utilized to thicken aqueous HPG solutions comprising 1.5 g HPG and 336 g $H_2O$. Firm gels were obtained with each agent upon additions of 0.5 g and 0.3 g to separate HPG solutions, with the use of 0.3 g appearing to yield a more flexible, less brittle gel than with the use of 0.5 g thickening agent.

As indicated by the preceding examples, factors relating to the stabilization of some of the compounds according to the invention in order to maintain them in liquid form must be considered. Although zirconates according to the invention would be utile as solids (*e.g.,* for ease of handling and shipment to point-of-use), the majority of users of resource recovery fluids have equipment and processes geared to the immediate utilization of thickening agents in liquid form.

Stabilization of the bis(n-propoxy) bis(2,4-pentanedionato)zirconium (IV) compound was most effectively achieved, as indicated in *Example 1,* through addition of chloroform following the removal of some reaction solvent. When chloroform is used, best results at stabilization have been achieved with the addition of about 10 wt.% chloroform based on the weight of the reaction product solution. With respect to the bis(n-butoxy) bis(2,4-pentanedionato)zirconium (IV) of *Example 2,* it was found that a stabilized product could be achieved in the first instance by ensuring that the molar ratio of the tetraalkyl zirconate to the ß-diketone reactant was 1:1.5 to less than 2, *i.e.,* 1:1.5 to <2. A reaction ratio of 1:1.95, for example, yielded a stabilized liquid requiring no additional steps to prevent crystallization of the product.

Where a solid product is desired, the preferred reactant ratios are from 1:2 to about 1:6 (zirconate to ß-diketone).

The composition of solid products obtained according to the process of the invention is unclear. Where the ß-diketone reactant is acetylacetone and the other reactant a tetraalkyl zirconate, for example, such as tetra (n-propyl) or tetra(n-butyl) zirconate, a spectrum of products that vary with the molar ratios of the reactants were observed via their physical state and their activities as thickening agents for aqueous polysaccharide solutions such as hdyroxypropylguar (HPG) solutions. If the molar ratio of zirconate:ß-diketone is less than 1:1.5, the reaction product of the invention will be liquid and tend to merely hydrolyze in the solution and have relatively little or no thickening effect. With a molar ratio of from 1:1.5 to less than 2, the product of the invention will likely be liquid, may or may not require stabilization efforts to maintain it in liquid form, and will be a utile thickening agent for aqueous polysaccharide solutions. If a molar ratio of 1:2 or more is used, the reaction product will likely be a solid which is also an effective thickening agent for aqueous polysaccharide solutions.

The bisalkyl bis(ß-diketone)zirconates of this invention are effective thickening agents for polysaccharides in aqueous solutions, most notably polymers of guar gum and cellulose derivatives. Particularly preferred are hydratable polysaccharides such as galactomannan gums, and derivatives thereof, and cellulose derivatives. Examples of such compounds are guar gum, locust bean gum, karaya gum, sodium carboxymethylguar, hydroxyethylguar, sodium carboxymethylhydroxyethylguar, hydroxypropylguar, sodium carboxymethylhyroxymethylcellulose, sodium carboxymethylhydroxyethylcellulose and hydroxyethylcellulose. The hydroxyethylcellulose derivatives used as gelling agents should be those having from 0.5 to 10 moles of ethylene oxide per anhydroglucose unit. The most preferred gelling agent for use in accordance with the present invention is hydroxypropylguar (HPG).

Thickened aqueous solutions as described herein are particularly useful in the oil and gas industry as drilling muds, sands consolidations fluids and fracturing fluids.

The subject zirconates exhibit enhanced temperature stability when used to thicken galactomannan gums as compared to results with prior art thickening agents, such as the titanates. Thus when utilized as thickening agents for fluids used in resource recovery operations, zirconates according to the invention may be used in high temperature environments that would destabilize titanate thickening agents. This consideration takes on special significance as recovery operations, especially in the area of oil drilling, are directed to less-readily available sources of materials. The zirconates may, for example, be used to thicken

galactomannan gums for use in fracturing oil wells that are drilled deeper and therefore present a hotter environment for fracturing. Although not yet verified, it is believed that the ΔH of the Zr-O bond being greater than that for the Ti-O bond accounts for this enhanced temperature stability, as well as the fact that the most common coordination numbers for zirconium and titanium are respectively 8 and 6.

Example 6

To 167 g hydrated hydroxypropylguar in a stirred aqueous solution with an organic acid, sodium bicarbonate, a surfactant and a bactericide (this HPG formulation is commercially available as NWP-12 from NOWSCO, Inc., Houston, Texas and was here used in a concentration of 5.9 g/l was added 0.5 g of the *Example 2* compound, The solution thickened after approximately 7.6 seconds. Thickening was determined to have occurred as of the onset of the Weisenberg effect, *i.e.,* when the vortex of the stirred solution closed and the solution would begin to climb the stirrer shaft.

Example 7

The procedure of *Example 6* was repeated, except that 0.5 g of the *Example 2* compound was added to 169 g HPG in the indicated solution. The NOWSCO HPG solution increased from an original viscosity of 510 mPa.s to 144,000 mPa.s in 10 seconds.

**Claims**

1. The use of compounds having the structure

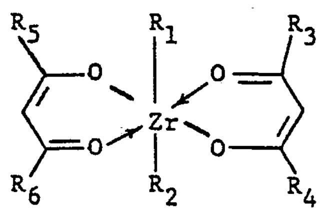

where $R_1$ and $R_2$ are individually selected from alkoxy having from 1 to 8 carbon atoms; aryloxy having from 6 to 12 carbon atoms; and

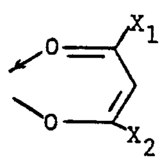

;

and $X_1$, $X_2$ and $R_3$ to $R_6$ are individually selected from alkyl having from 1 to 8 carbon atoms and aryl having from 6 to 12 carbon atoms, as thickening agents for aqueous polysaccharide solutions.

2. The use of compounds according to claim 1 wherein $R_1 = R_2$ = propoxy as thickening agents for aqueous polysaccharide solutions.

3. The use of compounds according to claim 1 wherein $R_1 = R_2$ = butoxy as thickening agents for aqueous polysaccharide solutions.

4. The use of compounds according to claim 1 wherein $R_3 = R_4 = R_5 = R_6$ = methyl as thickening agents for aqueous polysaccharide solutions.

5. The use of bis(n-propoxy)bis(2,4-pentanedionato) zirconium(IV) and bis(n-butoxy)bis(2,4-pentanedionato) zirconium(IV) as thickening agents for aqueous polysaccharide solutions.

6. A liquid product for use as a thickening agent for aqueous polysaccharide solutions, said product comprising bis(n-propoxy)bis(2,4-pentanedionato)zirconium(IV) and chloroform.

7. A liquid product for use as a thickening agent for aqueous polysaccharide solutions, said product comprising bis(n-butoxy)bis(2,4-pentanedionato)zirconium(IV) and tetra(n-butyl)zirconate.

8. A method of making a composition for use as a thickening agent for aqueous polysaccharide solutions, said method comprising the steps of admixing tetra(n-propyl)zirconate and acetylacetone in an organic solvent and subsequently adding a second organic solvent to the product of said zirconate-acetylacetone admixture to maintain said product in solution.

9. A method of making a composition for use as a thickening agent for aqueous polysaccharide solutions, said method comprising the step of admixing tetra(n-butyl)zirconate and acetylacetone in an organic solvent in a molar ratio of from 1:1.5 to <2 (zirconate:acetylacetone) to maintain the product of said admixture in solution.

**Patentansprüche**

1. Verwendung von Verbindungen der Strukturformel

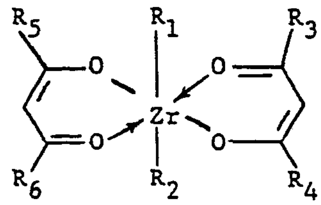

in der $R_1$ und $R_2$ für Alkoxygruppen mit 1 bis 8 C-Atomen, Aryloxygruppen mit 6 bus 12 C-Atomen oder die Gruppierung

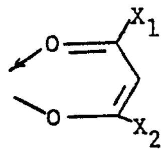

;

stehen und $X_1$, $X_2$, $R_3$ bis $R_6$ für Alkylgruppen mit 1 bis 8 C-Atomen oder Arylgruppen mit 6 bis 12 C-Atomen stehen, als Verdrickungsmittel für wäßrige Polysaccharid-Lösungen.

2. Verwendung der Verbindungen gemäß Anspruch 1 mit $R_1 = R_2 =$ Propoxy als Verdickungsmittel für wäßrige Polysaccharid-Lösungen.

3. Verwendung der Verbindungen gemäß Anspruch 1 mit $R_1 = R_2 =$ Butoxy als Verdickungsmittel für wäßrige Polysaccharid-Lösungen.

4. Verwendung der Verbindungen gemäß Anspruch 1 mit $R_3 = R_4 = R_5 = R_6 =$ Mehtyl als Verdickungsmittel für wäßrige Polysaccharid-Lösungen.

5. Verwendung von Bis)(n-propoxy)-bis(2,4-pentandionato)zirkonium(IV) und Bis-(n-butoxy)-bis(2,4-pentandionato)-zirkonium(IV) als Verdickungsmittel für wäßrige Polysaccharid-Lösungen.

6. Als Verdickungsmittel für wäßrige Polysaccharid-Lösungen verwendbares flüssiges Produkt, das Bis-(n-propoxy)-bis(2,4-pentandionato)-zirkonium und Chloroform enthält.

7. Als Verdickungsmittel für wäßrige Polysaccharid-Lösungen verwendbares flüssiges Produkt, das Bis-(n-butoxy)-bis(2,4-pentandionato)-zirkonium und Tetra-(n-butyl)-zirkonat enthält.

8. Verfahren zur Herstellung einer als Verdickungsmittel für wäßrige Polysaccharid-Lösungen dienenden Verbindung, dadurch gekennzeichnet, daß man ein Gemisch aus Tetra-(n-propyl)-zirkonat und Acetylaceton in einem organischen Lösungsmittel herstellt und daraufhin ein zweites organisches Lösungsmittel diesem Zirkonat-Acetylaceton-Gemisch hinzufügt, um dieses Gemisch in Lösung zu halten.

9. Verfahren zur Herstellung einer als Verdickungsmittel für wäßrige Polysaccharid-Lösungen dienenden Verbindungen, dadurch gekennzeichnet, daß man ein Gemisch aus Tetra-(n-butyl)-zirkonat und Acetylaceton im Molverhältnis von 1:1,5 bis 2 mit einem organischen Lösungsmittel vermischt, um das erhaltene Gemisch in Lösung zu halten.

**Revendications**

1. Utilisation de composés ayant la structure:

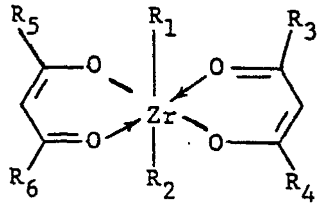

dans laquelle $R_1$ et $R_2$ sont choisis individuellement à partir d'un alcoxy ayant 1 à 8 atomes de carbone, d'un aryloxy ayant à 12-atomes de carbone et

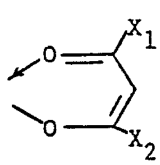

;

tandis que $X_1$, $X_2$ et $R_3$ à $R_6$ sont choisis individuellement parmi un alcoyle ayant 1 à 8 atomes de carbone et un aryle ayant 6 à 12 atomes de carbone, comme agents épaississants pour solutions aqueuses de polysaccharides.

2. Utilisation des composés selon la revendication 1, dans laquelle $R_1 = R_2$ = propoxy comme agents épaississants pour solutions aqueuses de polysaccharides.

3. Utilisation des composés selon la revendication 1, dans laquelle $R_1 = R_2$ = butoxy comme agents épaississants pour solutions aqueuses de polysaccharides.

4. Utilisation des composés selon la revendication 1, dans laquelle $R_3 = R_4 = R_5 = R_6$ = méthyle comme agents épaississants pour solutions aqueuses de polysaccharides.

5. Utilisation de bis(n-propoxy)bis(2,4-pentanedionato)zirconium(IV) et de bis(n-butoxy)bis(2,4-pentanedionato)zirconium(IV) comme agents épaississants pour solutions aqueuses de polysaccharides.

6. Produit liquide à l'usage d'agent épaississant pour solutions aqueuses de polysaccharides, ce produit comprenant du bis(n-propoxy)bis(2,4-pentanedionato)zirconium (IV) et du chloroforme.

7. Produit liquide à l'usage d'agents épaississants pour solutions aqueuses de polysaccharides, ce produit comprenant du bis(n-butoxy)bis(2,4-pentanedionato)zirconium (IV) et du zirconate de tétra-(n-butyle).

8. Procédé de fabrication d'une composition à l'usage d'agent épaississant pour solutions aqueuses de polysaccharides, ce procédé comprenant les stades de mélange de zirconate de tétra(n-propyle) et d'acétylacétone dans un solvant organique et d'addition consécutive d'un second solvant organique au produit de ce mélange zirconate-acétylacétone pour maintenir ce produit en solution.

9. Procédé de fabrication d'une composition à l'usage d'agent d'épaississant pour solutions aqueuses de polysaccharides, ce procédé comprenant le stade de mélange de zirconate de tétra(n-butyle) et d'acétylacétone dans un solvant organiques dans un rapport molaire de 1:1.5 à $<2$ (zirconate/acétylacétone) pour maintenir le produit de ce mélange en solution.